# EUROPEAN PATENT SPECIFICATION

(11) **EP 1 272 219 B1**
(45) Date of publication and mention of the grant of the patent: **13.12.2006**
(21) Application number: 01916044.9
(22) Date of filing: 27.03.2001
(51) Int. Cl.: A61K 45/06, A61K 31/195, A61K 31/40, A61K 31/505, A61P 3/06, A61P 9/10

(54) **NEW COMBINATION OF A BETABLOCKER AND A CHOLESTEROL-LOWERING AGENT**
NEUE KOMBINATION VON EINEM BETA-BLOCKER UND EINEM CHOLESTERIN-SENKENDEN MITTEL
NOUVELLE COMBINAISON D'UN BETABLOQUANT ET D'UN HYPOCHOLESTEROLEMIANT

(30) Priority: 03.04.2000 SE 0001188; 22.06.2000 SE 0002352
(43) Date of publication of application: 08.01.2003
(73) Proprietor: AstraZeneca AB, 151 85 Södertälje (SE)
(72) Inventor: BONDJERS, Göran, S-443 34 Lerum (SE); WIKLUND, Olov, S-431 69 Mölndal (SE); WIKSTRAND, John, S-431 83 Mölndal (SE)
(86) International application number: PCT/SE2001/000663
(87) International publication number: WO 2001/074394

(56) References cited:
- WO-A1-00/38725
- WO-A1-97/38694
- WO-A1-98/02357
- WO-A1-99/11260
- G. BERGLUND ET AL.: 'Low dose metoprolol and fluvastatin slow progression of atherosclerosis: Main result from BCAPS' ATHEROSCLEROSIS vol. 151, no. 1, June 2000, page 4, ABSTRACT NO. MOW3:3, XP002941316
- B. HEDBLAD J. ET AL.: 'Low dose metroprolol and fluvastatin in silent atherosclerotic disease: Metrobolic and adverse effects in BCAPS' ATHEROSCLEROSIS vol. 151, no. 1, June 2000, page 24, ABSTRACT NO. MOP7:W3, XP002941317
- C.J. EAGLES ET AL.: 'The effects of combined treatment with beta-I-selective receptor antagonists and lipid-lowering drugs on fat metabolism and measures of fatigue during moderate intensity excecise: a placebo-controlled study in healthy subjects' BR. J. CLIN. PHARMACOL. vol. 43, no. 3, 1997, pages 291 - 300, XP002941319
- OLAV PER FOSS ET AL.: 'Treatment of hypertensive and hypercholesterolaemic patients in general practice' SCAND. J. PRIMARY HEALTH CARE vol. 17, 1999, pages 122 - 127, XP002946315
- JAMES L. POOL ET AL.: 'Lovastatin and coadministred antihypertensive/cardiovascular agents' HYPERTENSION vol. 19, no. 3, March 1992, pages 242 - 248, XP002941325
- S. WITCHITZ O. ET AL.: 'Effets compares du captopril et de l'atenolol sur le metabolisme lipidique chez des patients hypertendus hypercholesterolemiques traites par pravastatine' LA PRESSE MEDICALE vol. 25, no. 40, 21 December 1996, pages 2013 - 2016, XP002941320
- WILLIAM R. GARNETT: 'Interactions with hydroxymethylglutaryl-coenzyme A reductase inhibitors' AM. J. HEALTH-SYST. PHARM. vol. 52, 01 August 1995, pages 1639 - 1645, XP002941324

## Description

### FIELD OF THE INVENTION

The present invention relates to pharmaceutical formulations comprising a betablocker and a cholesterol-lowering agent, particularly a HMG-CoA reductase inhibitor, in admixture with a pharmaceutically acceptable adjuvant, diluent or carrier, as well as a kit of parts, and the use of the formulations for the prophylactic or therapeutic treatment of atherosclerosis, particularly in patients suffering from hyperlipidemia, including hypercholesterolemia.

### BACKGROUND OF THE INVENTION

There is a constant need for new medications to further reduce the risk of atherosclerotic disease. This is especially true for patient populations at a higher risk than the population at large, e,g, patients suffering from hypercholesterolemia and hyperlipoproteinemia.

Various pharmaceuticals, such as the group known as statins, are known to lower the concentration of total serum cholesterol as well as influence the ratio between the concentration of the positive high-density lipid (HDL) cholesterol and the negative low-density lipid (LDL) cholesterol.

Betablockers on the other hand are known to have a positive influence on various cardiovascular disease, primarily hypertension, whereas physicians have generally avoided prescription of betablockers for patients with hypercholesterolemia.

The use of a combination of betablockers and cholesterol-lowering agents for the prophylactic and therapeutic treatment of patients suffering from, or susceptible to, atherosclerosis, hypercholesterolemia or hyperlipoproteinemia has not been disclosed previously.

The administration of combinations of β₁ selective blockers and lipid-lowering drugs to healthy volunteers, in order to observe the effects on fat metabolism, ammonia levels and the perception of effort during exercise, was disclosed in Br. J. Clin Pharmacol 1997 vol 43, no 3 pages 291-300. The combinations studied were 1) metoprolol (controlled release) and fluvastatin 2) metoprolol (controlled release) and bezafibrate 3) atenolol (normal release) and fluvastatin and 4) atenolol (normal release) and bezafibrate. The paper concluded that that these four combinations each caused significant reductions in fat metabolism, increased plasma ammonia concentrations and raised the perception of exercise. Combination 1) had the least adverse effect but the formulation difference was thought to be a significant factor in explaining the differences observed with respect to combination 3).

The effects of a combination of pravastatin and atenolol on hypertensive and hypercholesterolaemic patients was reported in Scand. J. Print Health Care 1999, vol 17 122-127. The conclusion was that the effect of atenolol was not influenced by the concurrent administration of pravastatin and *vice versa.* However, lifestyle intervention was also a feature of this study and therefore the conclusions which can be drawn from this study are not clear.

A *post hoc* analysis of patients who had been treated with lovastatin and who were also receiving antihypertensive medication including β₁ adrenergic receptor blockers was reported in Hypertension, 1992, Vol.19,3 242. The paper concluded that, subject to a number of limitations, there was no evidence for an attenuation of the lovastsatin-induced changes in lipids and lipoproteins or an alteration in the safety profile of lovastatin when administered concurrently with commonly used antihypertensive agents.

It was concluded in Presse Med Volume 1996, vol 25, no.40, 2013-2016 that the effect of the β₁ adrenergic receptor blocker atenolol was not diminished in combination with pravastatin. However, the effect of pravastatin on lipid metabolism was more favourable when pravastatin was combined with the angiotensin converting enzyme inhibitor captopril rather than with atenolol.
WO98/02357 discloses a carton for carrying pharmaceutically active substances or combinations thereof. One such combination mentioned is the combination of a beta blocker, such as metoprolol or isosorbidmononitrate, and a lipid lowering substance, such as fluvastatin. This application does not disclose any data concerning the effects of such a combination.
WO 99/11260 discloses a combination of atorvastatin and an antihypertensive agent. No data are disclosed in this application.
WO97/38694 discloses a combination of an HMG-CoA reductase (3-hydroxy-3-methylglutaryl coenzyme A reductase) inhibitor in combination with folic acid in combination with a drug selected from a range of other types of drug including beta blockers.
WO 00/38725 discloses combinations of an ileal bile transport inhibitor and a range of other types of drug including antihypertensive drugs for example beta blockers. No data are presented.

### SUMMARY OF THE INVENTION

The present invention relates to pharmaceutical combinations containing a betablocker and a cholesterol-lowering agent, pharmaceutical formulations containing a betablocker and a cholesterol-lowering agent in admixture with a pharmaceutically acceptable adjuvant, diluent or carrier, as well as the use of the formulations for the prophylactic or therapeutic treatment of atherosclerosis, hypercholesterolemia and hyperlipoproteinemia, wherein the betablocker is metoprolol and the HMG-CoA reductase inhibitor is (E)-7-[4-(4-fluorophenyl)-6-isopropyl-2-[methyl(methylsulfonyl)-amino]-pyrimidin-5-yl](3R,5S)-3,5-dihydroxyhept-6-enoic acid.

The invention further relates to a kit of parts of vessels containing the betablocker and the cholesterol-lowering agent and instructions for the administration of the betablocker and cholesterol-lowering agent to a patient for which such administration is necessary or advantageous.

The invention also relates to a kit of parts of formulations containing the betablocker and the cholesterol-lowering agent each in admixture with a pharmaceutically acceptable adjuvant, diluent or carrier.

### DETAILED DESCRIPTION OF THE INVENTION

It has surprisingly been found that, compared to the use of a cholesterol-lowering agent as a monotherapy, the present invention comprising a combination of a cholesterol-lowering agent and a beta-blocker has an enhanced anti-atherosclerotic effect, particularly in patients suffering from hypercholesterolemia or hyperlipoproteinemia.

In one aspect, the present invention thus relates to pharmaceutical combinations comprising a betablocker and a HMG-CoA reductase inhibitor wherein the betablocker is metoprolol, or a pharmaceutically acceptable salt or solvate thereof, or a solvate of such a salt; and the HMG-CoA reductase inhibitor is (E)-7-[4-(4-fluorophenyl)-6-isopropyl-2-[methyl(methylsulfonyl)amino]-pyrimidin-5-yl](3R,5S)-3,5-dihydroacyhept-6-enoic acid or a pharmaceutically acceptable salt or solvate thereof, or a solvate of such a salt.

In another aspect, the present invention relates to a pharmaceutical formulation comprising a betablocker and a HMG-CoA reductase inhibitor wherein the betablocker is metoprolol or a pharmaceutically acceptable salt or solvate thereof, or a solvate of such a salt and the HMG-CoA reductase inhibitor is (E)-7-[4-(4-fluorophenyl)-6-isopropyl-2-[methyl(methylsulfonyl)-amino]-pyrimidin-5-yl](3R,5S)-3,5-dihydroxyhept-b-enoic acid or a pharmaceutically acceptable salt or solvate thereof, or a solvate of such a salt in admixture with a pharmaceutically acceptable adjuvant, diluent or carrier.

A further aspect of the present invention relates to kits of parts comprising:
(i) a vessel containing metoprolol or a pharmaceutically acceptable salt or solvate thereof, or a solvate of such a salt and
(ii) a vessel containing a HMG-CoA reductase inhibitor which is (E)-7-[4-(4-fluorophenyl)-6-isopropyl-2-[methyl(methylsulfonyl)amino]-pyrimidin-5-yl](3R,5S)-3,5-dihydroxyhept-6-enoic acid or a pharmaceutically acceptable salt, especially sodium or calcium, or solvate thereof, or a solvate of such a salt
and instructions for the sequential, separate or simultaneous administration of the betablocker and a HMG-CoA reductase inhibitor to a patient for which such administration is necessary or advantageous.

Another aspect of the invention relates to kits of parts comprising:
(i) a pharmaceutical formulation containing metoprolol or a pharmaceutically acceptable salt or solvate thereof, or a solvate of such a salt in admixture with a pharmaceutically acceptable adjuvant, diluent or carrier; and
(ii) a pharmaceutical formulation containing (E)-7-[4-(4-fluorophenyl)-6-isopropyl-2-[methyl(methylsulfonyl)amino]-pyrimidin-5-yl](3R,5S)-3,5-dihydroxyhept-6-enoic acid or a pharmaceutically acceptable salt, in admixture with a pharmaceutically acceptable adjuvant, diluent or carrier;
wherein the betablocker and the cholesterol-lowering agent are each provided in a form that is suitable for administration in conjunction with the other.

By "administration in conjunction with", we include that respective formulations comprising a betablocker and a cholesterol-lowering agent are administered, simultaneously, separately or sequentially, over the course of treatment of the relevant condition, which condition may be acute or chronic. Particularly, the term includes that the two formulations are administered (optionally repeatedly) sufficiently closely in time for there to be a beneficial effect for the patient, that is greater, over the course of the treatment of the relevant condition, than if either of the two formulations are administered (optionally repeatedly) alone, in the absence of the other formulation, over the same course of treatment. It must, however, be emphasized that betablockers have never been used previously to give an anti-atherosclerotic effect e.g. in patients suffering from hypercholesterolemia or hyperlipoproteinemia, Determination of whether a combination provides a greater beneficial effect in respect of, and over the course of treatment of, a particular condition, will depend upon the condition to be treated or prevented, but may be achieved routinely by the person skilled in the art.

Thus, the term "in conjunction with" includes that one or other of the two formulations may be administered (optionally repeatedly) prior to, after, or at the same time as, administration with the other component. When used in this context, the terms "administered simultaneously" and "administered at the same time as" include that individual doses of a betablocker and a cholesterol-lowering agent are administered within 48 hours, e.g. 24 hours, of each other.

A still further aspect of the invention relates to the use of pharmaceutical formulations, comprising:
(i) a betablocker which is metoprolol or a pharmaceutically acceptable salt or solvate thereof, or a solvate of such a salt and
(ii) a cholesterol-lowering agent which is (E)-7-[4-(4-fluorophenyl)-6-isopropyl-2-[methyl(methylsulfonyl)-amino]-pyrimidin-5-yl](3R,5S)-3,5-dihydroxyhept-6-enoic acid or a pharmaceutically acceptable salt or solvate thereof, or a solvate of such a salt
in admixture with a pharmaceutically acceptable adjuvant, diluent or carrier, in the manufacture of a medicament for the prophylactic or therapeutic treatment of atherosclerosis.

In the present invention the betablocker is metoprolol or pharmaceu-tically acceptable salts or solvates thereof, or solvates of such salts. Particularly, the betablocker is metoprolol succinate (disclosed in US 5,001,161), metoprolol tartrate or metoprolol fumarate.

The pure enantiomers, racemic mixtures and unequal mixtures of two enantiomers of a betablocker are within the scope of the present invention. It should also be understood that all the diastereomeric forms possible are within the scope of the invention.

In the present application, the term "betablocker" also include chemical modifications of the betablockers, such as esters, prodrugs and metabolites, whether active or inactive.

### Cholesterol-lowering agents

The cholesterol-lowering agents referred to in this application are limited to inhibitors of HMG-CoA reductase (3-hydroxy-3-methylghrtaryl coenzyme A reductase) particularly (E)-7-[4-(4-fluorophenyl)-6-isopropyl-2-[methyl(methylsulfonyl)-amino]-pyrimidin-5-yl](3R,5S)-3,5-dihydroxyhept-6-enoic acid, [also known as (E)-7-[4-(4-fluorophenyl)-6-isopropyl-2-[*N*-methyl-*N*-(methylsulfonyl)-amino]pyrimidin-5-yl](3R,5S)-3,5-dihydroxyhept-6-enoic acid] or a pharmaceutically acceptable salt or solvate thereof, or a solvate of such a salt. The compound (E)-7-[4-(4-fluorophenyl)-6-isopropyl-2-[methyl-(methylsulfonyl)-amino]-pyrimidin-5-yl](3R,5S)-3,5-dihydroxyhept-6-enoic acid, and its calcium and sodium salts are disclosed in European Patent Application, Publication No. EP-A-0521471, and in Bioorganic and Medicinal Chemistry, (1997), 5(2), 437-444. This latter statin is now known under its generic name rosuvastatin.

### Pharmaceutical formulations

The present invention relates to pharmaceutical compositions containing a betablocker and a cholesterol-lowering agent as active ingredients.

Preferred combinations of a betablocker and a cholesterol-lowering agent are those where the betablocker is metoprolol particularly metoprolol succinate or metoprolol tartrate, and the cholesterol-lowering agent is a statin with the chemical name (E)-7-[4-(4-fluorophenyl)-6-isopropyl-2-[methyl(methylsulfonyl)amino]-pyrimidin-5-yl](3R,5S)-3,5-dihydroxyhept-6-enoic acid, or a pharmaceutically acceptable salt, especially the calcium and sodium salts, or solvate thereof or a solvate of such a salt.

A most preferred combination comprises metoprolol, particularly metoprolol succinate or metoprolol tartrate, and (E)-7-[4-(4-fluorophenyl)-6-isopropyl-2-[methyl(methylsulfonyl)-amino]-pyrimidin-5-yl](3R,5S)-3,5-dilydroxyhept-6-enoic acid, or a pharmaceutically acceptable salt, especially the calcium and sodium salts, or solvate thereof, or a solvate of such a salt.

In the present invention, the formulation and/or kits of parts may comprise two or more betablockers in combination with a cholesterol-lowering agent, two or more cholesterol-lowering agents in combination with a betablocker or any combination thereof.

For clinical use, the betablocker and the cholesterol-lowering agent are formulated into a pharmaceutical formulation for oral, intravenous, subcutaneous, tracheal, bronchial, intranasal, pulmonary, transdermal, buccal, rectal, parenteral or some other mode of administration. The pharmaceutical formulation contains the betablocker and the cholesterol-lowering agent in admixture with a pharmaceutically acceptable adjuvant, diluent or carrier.

The total amount of active ingredients suitably is in the range of from about 0.1 % (w/w) to about 95 % (w/w) of the formulation, suitably from 0.5 % to 50 % (w/w) and particularly from 1 % to 25 % (w/w).

The molar ratio between the betablocker and the cholesterol-lowering agent may be in the range of from about 1000:1 to about 1:1000. The molar ratio between the betablocker and the cholesterol-lowering agent lies suitably in the range of from 300:1 to 1:300, and particularly from 50:1 to 1:50.

In the preparation of the pharmaceutical formulations of the present invention the active ingredients may be mixed with solid, powdered ingredients, such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or pressed into tablets.

The active ingredients may be separately premixed with the other non-active ingredients, before being mixed to form a formulation. The active ingredients may also be mixed with each other, before being mixed with the non-active ingredients to form a formulation.

Soft gelatine capsules may be prepared with capsules containing a mixture of the active ingredients of the invention, vegetable oil, fat, or other suitable vehicle for soft gelatine capsules. Hard gelatine capsules may contain granules of the active ingredients. Hard gelatine capsules may also contain the active ingredients in combination with solid powdered ingredients such as lactose, saccharose, sorbitol, mannitol, potato starch, corn starch, amylopectin, cellulose derivatives or gelatine.

Dosage units for rectal administration may be prepared (i) in the form of suppositories which contain the active substance mixed with a neutral fat base; (ii) in the form of a gelatine rectal capsule which contains the active substance in a mixture with a vegetable oil, paraffin oil or other suitable vehicle for gelatine rectal capsules; (iii) in the form of a ready-made micro enema; or (iv) in the form of a dry micro enema formulation to be reconstituted in a suitable solvent just prior to administration.

Liquid preparations may be prepared in the form of syrups or suspensions, e.g. solutions or suspensions containing the active ingredients and the remainder consisting, for example, of sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents. Liquid preparations may also be prepared in the form of a dry powder to be reconstituted with a suitable solvent prior to use.

Solutions for parenteral administration may be prepared as a solution of a formulation of the invention in a pharmaceutically acceptable solvent. These solutions may also contain stabilizing ingredients, preservatives and/or buffering ingredients. Solutions for parenteral administration may also be prepared as a dry preparation to by reconstituted with a suitable solvent before use.

The dose of the compounds to be administered will depend on the relevant indication, the age, weight and sex of the patient and may be determined by a physician. The dosage will particularly be in the range of from 0.01 mg/kg to 10 mg/kg.

The typical daily dose of the active ingredients varies within a wide range and will depend on various factors such as the relevant indication, the route of administration, the age, weight and sex of the patient and may be determined by a physician. In general, oral and parenteral dosages will be in the range of 0.1 to 1,000 mg per day of total active ingredients.

For the cholesterol-lowering agent, any dose used in clinical practice may be used in the formulations and kits of parts of the present invention.

In the present invention, "a therapeutically effective total amount" relates to a total amount of the a betablocker and the cholesterol-lowering agent which when given in combination gives a therapeutic effect, even though each amount when given separately may be less than the therapeutically effective amount.

### Medical and pharmaceutical use

Also provided according to the present invention are formulations and kits of parts for use in medical therapy and particularly for use in the prophylactic or therapeutic treatment of atherosclerosis; the use of formulations of the present invention in the manufacture of medicaments for use in the prophylactic or therapeutic treatment of atherosclerosis, and methods of medical treatment or prophylaxis comprising the administration of a therapeutically effective total amount of a betablocker and a cholesterol-lowering agent to a patient suffering from, or susceptible to, atherosclerosis.

The betablocker and the cholesterol-lowering agent can be administered as a combined preparation for simultaneous, separate or sequential use in atherosclerosis therapy.
Furthermore, the betablocker can be administered prior to the administration of the cholesterol-lowering agent or vice versa.

The term 'medical therapy' as used herein is intended to include prophylactic, diagnostic and therapeutic regimens carried out in vivo or ex vivo on humans or other mammals.

The formulations of the invention are expected to be useful in prophylactic or therapeutic treatment of atherosclerosis, particularly in patients suffering from, or susceptible to hyperlipoproteinemia, and in particular hypercholesterolemia.

More particularly the formulations of the invention are expected to be useful in prevention of clinical events associated with the progression of atherosclerosis and/or acute vascular accidents related to atherosclerotic disease and plaque including but not limited to stroke, myocardial infarction (MI), congestive heart failure (CHF), cognitive decline, peripheral vascular disease, and renal dysfunction.

The formulations of the invention are furthermore expected to be useful in prophylactic or therapeutic treatment of cardiovascular complications in general, including, but not limited to, hypertension, diabetes mellitus, congestive heart failure (CHF), myocardial infarction (MI) including acute myocardial infarction (AMI), stroke, and mortality.

The following Example is intended to illustrate, but in no way limit the scope of the invention.

### EXAMPLE

A three-year placebo-controlled pilot study was designed to investigate the effect of the betablocker metoprolol on ultrasound-assessed atherosclerosis in patients with primary hypercholesterolemia on concomitant therapy with a cholesterol-lowering agent. More particularly, the trial was a prospective, randomised, double blind study, the betablocker was a controlled-release formulation of metoprolol succinate (metoprolol CR/XL) and the effect was assessed by measuring the intima-media thickness (IMT) in the carotid artery of the patients.

Intima-media thickness of the common carotid artery is commonly used as a surrogate variable for generalized atherosclerosis including coronary atherosclerosis (Wikstrand J, Wiklund O. Frontiers in cardiovascular science: Quantitative measurements of atherosclerotic manifestations in humans. Arterioscl Thromb. 1992; 12:114-119, and Grobbee DE, Bots ML. Carotid artery intima-media thickness as an indicator of generalized atherosclerosis. J. Int Med. 1994; 236:567-573). Accordingly, IMT of the common carotid artery was selected as the major endpoint of the study. Good quality images of the far wall of the straight part of the common carotid artery are easy to obtain, and IMT can me measured in nearly all subjects with good reproducibility (Schmidt C, Wendelhag I. How can the variability in ultrasound measurement of intima-media thickness be reduced? Studies of interobserver variability in carotid and femoral arteries. Clin Phys. 1999;1:45-55.).

The inclusion criteria were willingness to participate, 20-70 years of age, serum cholesterol > 6.5 mmol/L, low density lipid (LPL) cholesterol > 5.0 mmol/L. and serum triglycerides < 4.5 mmol/L.

Patients with hypercholesterolemia (n=129) were randomized at placebo-run run in, 62 to metoprolol CR/XL (AstraZeneca, Mölndal. Sweden) treatment (100 mg o.d.) and 67 to placebo. During the two-weeks of placebo run-in 15 subjects from the metoprolol CR/XL group and 11 subjects from the placebo group were withdrawn due to negative ultrasound examinations (n=3 and n=1, respectively), low total cholesterol, low LDL cholesterol or high triglyceride levels (n=6 and n=4, respectively), myocardial infarction (n=1 and n=0, respectively), nausea (n=1 and n=2, respectively), unwillingness to participate (n=4 and n=2, respectively) and other causes (n=0 and n=2. respectively).

After start of double-blind treatment 12 subjects from the metoprolol CR/XL group and 12 subjects from the placebo group were withdrawn due to myocardial infarction (n=2 and n=1, respectively), atria! fibrillation (n=2 and n=0. respectively), angina pectoris (n=1 and n=1, respectively), malignancy (n=1 and n=1, respectively), minor side effects (n=1 and n=5. respectively), given metoprolol CR/XL by other physician (n=0 and n=1, respectively), moved to other area (n=2 and n=0), compliance (n=1 and n=1, respectively) and unwillingness (n=2 and n=2, respectively).

Subsequently seventy-nine patients completed the study: 35 in the metoprolol CR/XL group and 44 in the placebo-group. The distribution of males and females in the groups were similar, 18 and 22 males and 17 and 22 females in the metoprolol CR/XL and placebo group, respectively. Likewise, the mean age was 60.6 and 59.6 for the metoprolol CR/XL and placebo group, respectively. The mean follow-up time was 2.97 years.

All analyses for the patients with hypercholesterolemia in the present example refers to the 79 subjects who completed the study. Two subjects in each group reduced the dose during follow-up from 100 mg to 50 mg (metoprolol CR/XL or placebo).

Total cholesterol, HDL cholesterol and heart rate decreased more in the metoprolol CR/XL group compared with the placebo group (p<0.05).

### Cholesterol-lowering therapy in the metoprolol CR/XL group and the placebo group during follow-up

Subjects were treated with statins before inclusion, 31% in the metoprolol CR/XL group and 27% in the placebo group. However, all lipid-lowering drugs were withdrawn prior to start of double-blind medication.

The majority of subjects in the metoprolol CR/XL group and in the placebo group were treated with statins at the 1-year follow-up (91% and 80%, respectively), at the 2-year follow-up (88% and 82%, respectively), and at the 3-year follow up (94% and 84%, respectively). Ninety-four percent of the subjects in the metoprolol CR/XL group and 89% of the subjects in the placebo group were at any time during the 3-year follow-up treated with statins. Subjects were also treated with cholestyramine at the 1-year follow-up (23% and 30%, respectively), at the 2-year follow-up (26% and 23%, respectively), and at the 3-year follow-up (26% and 23%, respectively). All in the metoprolol CR/XL group and 96% of the subjects in the placebo group were treated with any lipid-lowering drug during follow-up.

During the 3-year follow-up LDL cholesterol was reduced by 44% in the metoprolol CR/XL group and by 38% in the placebo group (non-significant difference between groups).

### Carotid intima-media thickness (IMT) and lumen diameter in the three groups during follow-up

The intima-media thickness was defined as the distance between the leading edge of the lumen-intima interface of the far wall to the leading edge of the media-adventitia interface of the far wall. The lumen diameter was defined as the distance between the leading edges of the intima-lumen interface of the near wall and the lumen-intima interface of the far wall.

Mean values for common carotid LWT at the base-line examination and at the 3-vear follow-up were 0.91+0.23 and 0.87+0.17 mm, respectively, in the metoprolol CR/XL group; and 0.89+0.18 and 0.92+0.17 mm, respectively, in the placebo group (p<0.05 between groups for difference in change during follow-up). The regression coefficient for the yearly change in common carotid IMT was -0.010±0.06 mm and +0.012±0.04 for the metoprolol CR/XL group and the placebo group, respectively; and the 95% CI for the difference between the placebo group and the metoprolol CR/XL group was 0.0004 - 0.0444 mm (p<0.05).

Mean values for carotid bulb IMT at the base-line examination and at the 3-year follow-up were 1.42±0.46 and 1.34±0.41 mm, respectively, in the metoprolol CR/XL group: and 1.26±0.45 and 1.30±0.41 mm, respectively, in the placebo group (p=0.12 between groups for difference in change during follow-up).

Mean values for lumen diameter in the carotid artery at the base-line examination and at the 3-year follow-up were 6.30±0.78 and 6.15±0.85 mm, respectively, in the metoprolol CR/XL group: and 6.23±0.71and 6.14±0.69 mm, respectively, in the placebo group (no significant differences between groups).

Mean values for baseline common carotid IMT in subjects that were withdrawn from double-blind medication during follow-up were 0.86±0.11 and 0.89±0.17 mm in the metoprolol CR/XL group and the placebo group, respectively (no significant difference between groups).

## Claims

1. A pharmaceutical combination comprising a betablocker and a HMG-CoA reductase inhibitor wherein the betablocker is metoprolol or a pharmaceutically acceptable salt or solvate thereof, or a solvate of such a salt; and the HMG-CoA reductase inhibitor is (E)-7-[4-(4-fluorophenyl)-6-isopropyl-2-[Methyl(methylsulfonyl)-amino]-pyrimidin-5-yl](3R,5S)-3,5-dihydroxyhept-6-enoic acid or a pharmaceutically acceptable salt or solvate thereof, or a solvate of such a salt.

2. The pharmaceutical combination according to claim 1, wherein the betablocker is metoprolol succinate, metoprolol tartrate or metoprolol fumarate.

3. The pharmaceutical combination according to any previous claim wherein the HMG-CoA reductase inhibitor is (E)-7-[4-(4-fluorophenyl)-6-isopropyl-2-[methyl(methylsulfonyl)amino]pyrimidin-5-yl](3R,5S)-3,5-dihydroxyhept-6-enoic acid sodium or calcium salt, or a solvate thereof.

4. The pharmaceutical combination according to any previous claim, wherein the molar ratio between the betablocker and the cholesterol-lowering agent lies in the range of from 1000:1 to 1:1000.

5. A pharmaceutical combination according to claim 4 wherein the molar ratio between the betablocker and the cholesterol-lowering agent lies in the range of from 300:1 to 1:300.

6. A pharmaceutical formulation comprising a betablocker and a HMG-CoA reductase inhibitor wherein the betablocker is metoprolol or a pharmaceutically acceptable salt or solvate thereof, or a solvate of such a salt and the HMG-CoA reductase inhibitor is (E)-7-[4-(4-fluorophenyl)-6-isopropyl-2-[methyl(methylsulfonyl)-amino]-pyamidin-5-yl](3R,5S)-3,5-dihydroxyhept-6-enoic acid or a pharmaceutically acceptable salt or solvate thereof, or a solvate of such a salt in admixture with a pharmaceutically acceptable adjuvant, diluent or carrier.

7. The pharmaceutical formulation according to claim 6, wherein the betablocker is metoprolol succinate, metoprolol tartrate or metoprolol fumarate.

8. The pharmaceutical formulation according to either claim 6 or claim 7, wherein the HMG-CoA reductase inhibitor is (E)-7-[4-(4-fluorophenyl)-6-isopropyl-2-[methyl(methylsulfonyl)-amino]-pyrimidin-5-yl](3R,5S)-3,5-dihydroxyhept-6-enoic acid sodium or calcium salt, or a solvate thereof.

9. The pharmaceutical formulation according to any one of claims 6 to 8, wherein the molar ratio between the betablocker and the cholesterol-lowering agent lies in the range of from 1000:1 to 1:1000.

10. The pharmaceutical formulation according to claim 9 wherein the molar ratio between the betablocker and the cholesterol-lowering agent lies in the range of from 300:1 to 1:300.

11. A combination according to any one of claims 1 to 5 or a formulation according to any one of claims 6 to 10 for use in medical therapy.

12. The use of a formulation according to any one of claims 6 to 10, in the manufacture of a medicament for the prophylactic or therapeutic treatment of atherosclerosis.

13. The use according to claim 12, wherein the patient suffers from or, is susceptible to, hypercholesterolemia or hyperlipoproteinemia.

14. The use of a combination according to any one of claims 1 to 5 or a formulation according to any one of claims 6 to 10 for use in the manufacture of a medicament for the prophylactic or therapeutic treatment of a patient suffering from, or susceptible to, atherosclerosis.

## Patentansprüche

1. Pharmazeutische Kombination umfassend einen Betablocker und einen HMG-CoA-Reduktaseinhibitor, wobei es sich bei dem Betablocker um Metoprolol oder ein pharmazeutisch annehmbares Salz oder Solvat davon, oder ein Solvat eines derartigen Salzes, und bei dem HMG-CoA-Reduktaseinhibitor um (E)-7-[4-(4-Fluorphenyl)-6-isöpropyl-2-[methyl(methylsulfonyl)-amino]pyrimidin-5-yl](3R,5S)-3,5-dihydroxyhept-6-ensäure oder ein pharmazeutisch annehmbares Salz oder Solvat davon, oder ein Solvat eines derartigen Salzes, handelt.

2. Pharmazeutische Kombination nach Anspruch 1, wobei es sich bei dem Betablocker um Metoprololsuccinat, Metoprololtartrat oder Metoprololfumarat handelt.

3. Pharmazeutische Kombination nach einem der vorangehenden Ansprüche, wobei es sich bei dem HMG-CoA-Reduktaseinhibitor um ein Natrium- oder Calciumsalz von (E)-7-[4-(4-Fluorphenyl)-6-isopropyl-2-[methyl(methylsulfonyl)-amino]pyrimidin-5-yl](3R,5S)-3,5-dihydroxyhept-6-ensäure oder ein Solvat davon handelt.

4. Pharmazeutische Kombination nach einem der vorangehenden Ansprüche, wobei das Molverhältnis zwischen dem Betablocker und dem Cholesterin-senkenden Mittel im Bereich von 1000:1 bis 1:1000 liegt.

5. Pharmazeutische Kombination nach Anspruch 4, wobei das Molverhältnis zwischen dem Betablocker und dem Cholesterin-senkenden Mittel im Bereich von 300:1 bis 1:300 liegt.

6. Pharmazeutische Formulierung umfassend einen Betablocker und einen HMG-CoA-Reduktaseinhibitor, wobei es sich bei dem Betablocker um Metoprolol oder ein pharmazeutisch annehmbares Salz oder Solvat davon, oder ein Solvat eines derartigen Salzes, und bei dem HMG-CoA-Reduktaseinhibitor um (E)-7-[4-(4-Fluorphenyl)-6-isopropyl-2-[methyl(methylsulfonyl)-amino]pyrimidin-5-yl](3R,5S)-3,5-dihydroxyhept-6-ensäure oder ein pharmazeutisch annehmbares Salz oder Solvat davon, oder ein Solvat eines derartigen Salzes, handelt, in Abmischung mit einem pharmazeutisch annehmbaren Adjuvans, Verdünnungsmittel oder Trägermedium.

7. Pharmazeutische Formulierung nach Anspruch 6, wobei es sich bei dem Betablocker um Metoprololsuccinat, Metoprololtartrat oder Metoprololfumarat handelt.

8. Pharmazeutische Formulierung nach Anspruch 6 oder 7, wobei es sich bei dem HMG-CoA-Reduktaseinhibitor um ein Natrium- oder Calciumsalz von (E)-7-[4-(4-Fluorphenyl)-6-isopropyl-2-[methyl(methylsulfonyl)-amino]pyrimidin-5-yl](3R,5S)-3,5-dihydroxyhept-6-ensäure oder ein Solvat davon handelt.

9. Pharmazeutische Formulierung nach einem der Ansprüche 6 bis 8, wobei das Molverhältnis zwischen dem Betablocker und dem Cholesterin-senkenden Mittel im Bereich von 1000:1 bis 1:1000 liegt.

10. Pharmazeutische Formulierung nach Anspruch 9, wobei das Molverhältnis zwischen dem Betablocker und dem Cholesterin-senkenden Mittel im Bereich von 300:1 bis 1:300 liegt.

11. Kombination nach einem der Ansprüche 1 bis 5 oder Formulierung nach einem der Ansprüche 6 bis 10 zur Verwendung in der medizinischen Therapie.

12. Verwendung einer Formulierung nach einem der Ansprüche 6 bis 10 bei der Herstellung eines Arzneimittels zur prophylaktischen oder therapeutischen Behandlung von Athersklerose.

13. Verwendung nach Anspruch 12, wobei der Patient an Hypercholesterinämie oder Hyperlipoproteinämie leidet oder dafür prädispositioniert ist.

14. Verwendung einer Kombination nach einem der Ansprüche 1 bis 5 oder einer Formulierung nach einem der Ansprüche 6 bis 10 zur Verwendung bei der Herstellung eines Arzneimittels zur prophylaktischen oder therapeutischen Behandlung eines Patienten, der an Athersklerose leidet oder dafür prädispositioniert ist.

## Revendications

1. Combinaison pharmaceutique comprenant un bêtabloquant et un inhibiteur de la HMG-CoA réductase, dans laquelle le bêtabloquant est le métoprolol ou un sel ou un solvate pharmaceutiquement acceptable de celui-ci, ou un solvate d'un tel sel, et l'inhibiteur de la HMG-CoA réductase est l'acide (E) -7-[4-(4-fluorophényl)-6-isopropyl-2-[méthyl(méthylsulfonyl)-amino]pyrimidin-5-yl](3R,5S)-3,5-dihydroxyhept-6-enoïque ou un sel ou un solvate pharmaceutiquement acceptable de celui-ci, ou un solvate d'un tel sel.

2. Combinaison pharmaceutique selon la revendication 1, dans laquelle le bêtabloquant est du succinate de métoprolol, du tartrate de métoprolol ou du fumarate de métoprolol.

3. Combinaison pharmaceutique selon l'une quelconque des revendications précédentes, dans laquelle l'inhibiteur de la HMG-CoA réductase est le sel de sodium ou de calcium de l'acide (E) -7-[4-(4-fluorophényl)-6-isopropyl-2-[méthyl(méthylsulfonyl)amino]pyrimidin-5-yl](3R,5S)-3,5-dihydroxyhept-6-enoïque, ou un solvate de ce sel.

4. Combinaison pharmaceutique selon l'une quelconque des revendications précédentes, dans laquelle le rapport molaire entre le bêtabloquant et l'hypocholestérolémiant se trouve dans la plage allant de 1000:1 à 1:1000.

5. Combinaison pharmaceutique selon la revendication 4, dans laquelle le rapport molaire entre le bêtabloquant et l'hypocholestérolémiant se trouve dans la plage allant de 300:1 à 1:300.

6. Formulation pharmaceutique comprenant un bêtabloquant et un inhibiteur de la HMG-CoA réductase, dans laquelle le bêtabloquant est le métoprolol ou un sel ou un solvate pharmaceutiquement acceptable de celui-ci, ou un solvate d'un tel sel, et l'inhibiteur de la HMG-CoA réductase est l'acide (E)-7-[4-(4-fluorophényl)-6-isopropyl-2-[méthyl(méthylsulfonyl)-amino]pyrimidin-5-yl](3R,5S)-3,5-dihydroxyhept-6-enoïque ou un sel ou un solvate pharmaceutiquement acceptable de celui-ci, ou un solvate d'un tel sel dans un mélange avec un adjuvant, un diluant ou un excipient pharmaceutiquement acceptable.

7. Formulation pharmaceutique selon la revendication 6, dans laquelle le bêtabloquant est du succinate de métoprolol, du tartrate de métoprolol ou du fumarate de métoprolol.

8. Formulation pharmaceutique selon l'une quelconque des revendications 6 ou 7, dans laquelle l'inhibiteur de la HMG-CoA réductase est le sel de sodium ou de calcium de l'acide (E)-7-[4-(4-fluorophényl)-6-isopropyl-2-[méthyl(méthylsulfonyl)amino]pyrimidin-5-yl](3R,5S)-3,5-dihydroxyhept-6-enoïque, ou un solvate de ce sel.

9. Formulation pharmaceutique selon l'une quelconque des revendications 6 à 8, dans laquelle le rapport molaire entre le bêtabloquant et l'hypocholestérolémiant se trouve dans la plage allant de 1000:1 à 1:1000.

10. Formulation pharmaceutique selon la revendication 9, dans laquelle le rapport molaire entre le bêtabloquant et l'hypocholestérolémiant se trouve dans la plage allant de 300:1 à 1:300.

11. Combinaison selon l'une quelconque des revendications 1 à 5 ou formulation selon l'une quelconque des revendications 6 à 10 pour utilisation dans une thérapie médicale.

12. Utilisation de la formulation selon l'une quelconque des revendications 6 à 10, dans la fabrication d'un médicament pour le traitement prophylactique ou thérapeutique de l'athérosclérose.

13. Utilisation selon la revendication 12, dans laquelle le patient souffre, ou est susceptible de souffrir, d'hypercholestérolémie ou d'hyperlipoprotéinémie.

14. Utilisation d'une combinaison selon l'une quelconque des revendications 1 à 5 ou d'une formulation selon l'une quelconque des revendications 6 à 10 dans la fabrication d'un médicament pour le traitement prophylactique ou thérapeutique d'un patient atteint, ou susceptible d'être atteint, d'athérosclérose.
